Europäisches Patentamt

European Patent Office

Office européen des brevets

(11) Veröffentlichungsnummer: **0 312 495**
**A2**

(12) **EUROPÄISCHE PATENTANMELDUNG**

(21) Anmeldenummer: **88810677.0**

(22) Anmeldetag: **30.09.88**

(51) Int. Cl.⁴: **H 01 B 7/00**

---

(30) Priorität: **16.10.87 CH 4082/87**

(43) Veröffentlichungstag der Anmeldung:
**19.04.89 Patentblatt 89/16**

(84) Benannte Vertragsstaaten:
**AT BE CH DE ES FR GB GR IT LI LU NL SE**

(71) Anmelder: **INSTITUT STRAUMANN AG**
**CH-4437 Waldenburg (CH)**

(72) Erfinder: **Steinemann, Samuel G., Prof. Dr.**
**Chemin des Codoz 14**
**CH-1025 St. Sulpice (CH)**

(74) Vertreter: **Eder, Carl E.**
**Patentanwaltsbüro EDER AG Münchensteinerstrasse 2**
**CH-4052 Basel (CH)**

---

(54) **Elektrisches Kabel für die Durchführung mindestens einer Stimulation und/oder Messung in einem menschlichen oder tierischen Körper.**

(57) Das Kabel (1) weist mindestens ein Bündel (3) mit mindestens 100 Fasern (5) auf, deren Durchmesser weniger als 20 Mikrometer beträgt und die vorzugsweise aus einer Legierung bestehen, die als Basismetall Titan und noch mindestens eines der Metalle Niob, Tantal, Zirkon, Chrom, Molybdän, Eisen und Aluminium enthält. Das bzw. jedes Bündel (3) kann um die KabelLängsachse herumgedreht und/oder mit anderen Bündeln (3) verflochten sein. Ferner können die zum gleichen Bündel (3) gehörenden Fasern (5) eventuell verdrillt sein. Das bzw. jedes Bündel (3) und seine Fasern (5) sollen jedoch vorteilhafterweise derart verlaufen, dass die Länge des bzw. jedes Bündels (3) und auch die Länge jeder Faser (5) vorzugsweise höchstens 50 % und beispielsweise höchstens oder ungefähr 30 % länger ist als das Kabel (1). Die Fasern (5) sind bis zu sehr kleinen Krümmungsradien hinab biegbar, ohne dass Ermüdungsbrüche auftreten, wobei ein relativ niedriger, elektrischer Widerstand des Kabels (1) erzielt werden kann. An den Oberflächen der Fasern (5) ist ein Metalloxydschicht vorhanden, die die Fasern (5) zusätzlich zur normalerweise vorhandenen, gummielastischen Isolation (7) isoliert.

**Fig.1**

EP 0 312 495 A2

## Description

### Elektrisches Kabel für die Durchführung mindestens einer Stimulation und/oder Messung in einem menschlichen oder tierischen Körper

Die Erfindung betrifft ein elektrisches Kabel gemäss dem Oberbegriff des Anspruchs 1.

Kabel dieser Art werden unter anderem für Herzschrittmacher in menschliche und tierische Körper eingesetzt. Wie zum Beispiel im Aufsatz "Requirements of the ideal pacemaker leads", von A. Sinnaeve, R. Willems und R. Stroobandt, Seiten 47-55, in "Pacemaker leads", - herausgegeben von A. E. Aubert und H. Ector, Elsevier Science Publishes B.V. Amsterdam, 1985 erörtert ist, werden Kabel für Herzschrittmacher häufigen, mechanischen Beanspruchungen unterworfen, weil sie ja bei jedem Herzschlag und also ungefähr 37 Millionen Mal pro Jahr und zusätzlich noch durch die Atmungsbewegungen, Armbewegungen und andere Körperbewegungen bewegt und deformiert werden, wobei vor allem Biegungen, aber auch noch Torsionen und Dehnungen der Kabel stattfinden. Die Kabel sollten diesen mechanischen Beanspruchungen ohne Bruch standhalten, einen möglichst kleinen elektrischen Widerstand haben, gegenüber Blut und anderen Körperflüssigkeiten korrosionsbeständig und selbstverständlich auch möglichst dünn sein.

Viele derzeit gebräuchliche Kabel für Herzschrittmacher weisen mindestens einen im Querschnitt runden oder rechteckigen, d.h. bandförmigen Draht auf. Die Drähte können aus einer Platin- Iridium-Legierung, Silber, Gold, den unter den Bezeichnungen Elgiloy und MP35N im Handel erhältlichen, Kobalt, Chrom, Nickel und andere Komponenten aufweisenden Legierungen, eventuell Wolfram, rostfreiem Stahl und Aluminium oder Kupfer bestehen. Die Kabel sind üblicherweise aussen mit einer Isolation versehen und haben zum Teil auch noch einen Kern aus Isoliermaterial. Es sei diesbezüglich auf den bereits zitierten Aufsatz von Sinnaeve et al. und auch auf die EP-A-0 162 178 verwiesen. Bei den meisten bekannten Kabeln mit im Querscnitt runden Drähten haben diese typischerweise Durchmesser von mindestens oder ungefähr 0,1 mm. In der genannten EP-A-0 162 178 sind für die im Querschnitt runden Drähte Durchmesser von 20 bis 80 $\mu$m vorgeschlagen, wobei die tatsächlich verwendeten Drähte einen Durchmesser von 50 $\mu$m haben.

Die Drähte sind bei den meisten bekannten Kabeln einzeln oder wie zum Beispiel gemäss der bereits zitierten EP-A-0 162 178 bündelweise zu Wendeln gewickelt, wobei die Steigung der Drähte bzw. Drahtbündel im allgemeinen möglichst klein gemacht wird, sodass benachbarte Wendelwindungen zumindest annähernd aneinander anliegen. Dadurch kann die Biegbarkeit zwar gegenüber gerade verlaufenden Drähten verbessert werden, wobei aber beim Biegen mit kleinen Radien trotzdem je nach Typ noch mehr oder weniger häufig Ermüdungsbrüche auftreten können. Wenn zum Beispiel beim Kabel eines Herzschrittmachers infolge eines Drahtbruches ein vollständiger Stromunterbruch entsteht, kann dies den Tod des betreffenden Patienten verursachen. Die Bruchgefahr stellt daher einen grossen Nachteil dar.

Die aus der EP-A-0 162 178 bekannten Kabel, bei denen die Wendeln aus Bündel von je sieben miteinander verdrillten Drähten bestehen, sind bereits bis hinab zu etwa 1,5 mm betragenden Krümmungsradien ermüdungsfest. Es ist jedoch verhältnismässig schwierig und entsprechend aufwendig, Bündel von verdrillten Drähten zu Wendeln in der Art mehrgängiger Gewinde zu formen.

Bei allen bekannten Kabeln, bei denen die Drähte zu Wendeln mit mindestens annähernd aneinander anliegenden Windungen gewickelt sind, ist die abgewickelte Länge der Drähte ein Vielfaches der Länge des betreffenden Kabels. Dies wirkt sich ungünstig, nämlich stark erhöhend auf den elektrischen Widerstand aus. Dieser Nachteil lässt sich zwar teilweise dadurch reduzieren, das mindestens ein Teil der Drähte aus einem einen möglichst kleinen, spezifischen, elektrischen Widerstand aufweisenden Material, wie Platin-Iridium, Silber oder Kupfer hergestellt wird. Abgesehen davon, dass diese Materialien relativ ungünstige mechanische Eigenschaften haben, sind die beiden ersten verhältnismässig teuer und Kupfer wirkt, wenn es in Berührung mit Körpersäften oder -zellen gelangt, etwas toxisch.

Wenn Kabel mit mindestens einem Draht und einer diesen bzw. jeden Draht umhüllenden Kunststoffisolation in Körper von Menschen oder Tieren eingesetzt werden, besteht die Möglichkeit, dass die Isolation beim Einführen der Kabel oder später bei Bewegungen von die Kabel enthaltenden Körperteilen beschädigt wird. Dies kann, abgesehen vielleicht vom Aluminium, bei allen für bekannte Kabel verwendeten, vorgängig angegebenen Drahtmaterialien, zur Folge haben, dass die zugeführte elektrische Energie zumindest zum Teil schon vor dem Ende des Kabels in den Körper des Menschen oder Tieres austritt.

Zur Verbesserung des Verständnisses sollen jetzt noch einige geometrische und/oder physikalische Grössen sowie deren Beziehungen erörtert werden. Im folgenden wird unter dem kritischen Krümmungsradius $r_c$ der kleinste Krümmungsradius verstanden, mit dem ein Draht oder eine Faser eines Kabels biegbar ist, ohne dass ein Bruch stattfindet. Wenn ein Draht oder eine Faser das Flächenträgheitsmoment I und das Widerstandsmoment W hat und das den Draht bzw. die Faser bildende Material den Elastizitätsmodul E und bei Zugbeanspruchung die maximal zulässige Spannung $\sigma_z$ hat, kann man zeigen, dass der kritische Krümmungsradius gegeben ist durch die Formel:

$$r_c = \frac{E \quad I}{W \quad \sigma_z'} \qquad (1)$$

· Wenn ein Draht oder eine Faser im Querschnitt kreisförmig ist und einen Durchmesser d hat, ist W = 2l/d und der kritische Krümmungsradius wird:

$$r_c = \frac{E \quad d}{2 \quad \sigma_z} \qquad (2)$$

Der elektrische Widerstand R eines Leiters, der aus einem Material mit dem spezifischen, elektrischen Widerstand   besteht und die Länge 1 sowie die Querschnittsfläche A hat, beträgt

$$R = \frac{\varrho \quad 1}{A} \qquad (3)$$

Gemäss den Formeln (1) und (2) ist der kritische Krümmungsradius proportional zum Elastizitätsmodul sowie umgekehrt proportional zur zulässigen Spannung und für Drähte oder Fasern mit kreisförmigem Querschnitt ferner proportional zum Durchmesser d. Eine bei bekannten Kabeln häufig verwendete Platin-Iridium-Legierung mit 10 Gew.-% Iridium und dem Rest Platin hat einen verhältnismässig hohen, nämlich etwa 150 GPa betragenden Elastizitätsmodul und eine relative niedrige, nämlich etwa 0,3 GPa betragende, zulässige Spannung bei Zugbeanspruchungen. Wenn man diese Werte in die Formel (2) einsetzt und zum Beispiel annimmt, dass die Drähte einen kreisförmigen Querschnitt und einen Durchmesser von 0,1 mm haben, ergibt sich für den kritischen Krümmungsradius ein Wert von 25 mm. Beim Einsatz eines Kabels in einem Körper finden jedoch Biegungen weit viel kleineren Krümmungsradien statt. Die ebenfalls für die Herstellung von Kabeln verwendeten, hochfesten Kobalt-Legierungen haben zwar höhere zulässige Spannungen, aber noch höhere Elastizitätsmoduln als die Platin-Iridium-Legierung und sind zudem schwer zu dünnen Drähten formbar.

Es ist in der Supraleitertechnik bekannt, eine grössere Anzahl aus einer Titan-Niob-Legierung bestehende, verhältnismässig dicke Drähte in eine Kupfermatrix einzubetten und den so gebildeten Verbund-Rohling zu strecken und dadurch dünner zu machen, so dass ein Verbund-Leiter entsteht, der in eine Kupfermatrix eingebettete Titan-Niob-Drähte enthält, deren Durchmesser etwa 12 bis 13 Mikrometer beträgt. Diese Verbund-Leiter dienen jedoch nicht zum Einsetzen in menschliche oder tierische Körper und wären dazu auch gar nicht geeignet, weil der Aussendurchmesser der fertigen Verbund-Leiter in der Grösse von 1 mm liegt und die Leiter daher sehr steif sind und weil Kupfer, wie bereits ausgeführt, im Inneren eines Körpers toxische Wirkungen haben kann.

Der Erfindung liegt nun die Aufgabe zugrunde, ein elektrisches Kabel für die Durchführung mindestens einer Stimulation und/oder Messung in einem menschlichen oder tierischen Körper und normalerweise für die Durchführung mehrerer solcher Stimulationen und/oder Messungen, beispielsweise für einen Herzschrittmacher, zu schaffen, das Nachteile der bekannten Kabel behebt. Dabei soll insbesondere die Gefahr von Ermüdungsbrüchen, von elektrischen Lecks sowie von toxischen Wirkungen möglichst weitgehend eliminiert werden und trotzdem ein geringer elektrischer Widerstand und eine wirtschaftliche Herstellbarkeit ermöglicht werden.

Diese Aufgabe wird durch ein Kabel der einleitend genannten Art gelöst, das erfindungsgemäss durch die Merkmale des Anspruchs 1 gekennzeichnet ist. Vorteilhafte Weiterbildungen des Kabels gehen aus den vom Anspruch 1 abhängigen Ansprüchen hervor.

Die Erfindung betrifft ferner ein Verfahren zur Herstellung eines Kabels, wobei das Verfahren gemäss der Erfindung durch den kennzeichnenden Teil des Anspruchs 10 gekennzeichnet ist. Vorteilhafte Ausgestaltungen des Verfahrens ergeben sich aus dem vom diesem Anspruch abhängigen Ansprüchen.

Das Kabel ist dazu vorgesehen, vollständig oder eventuell nur teilweise in einen menschlichen oder tierischen Körper eingesetzt zu werden und einen Teil einer Stimulations- und/oder Messeinrichtung zu bilden. Das Kabel ist beispielsweise zur Herzstimulation, d.h. zur Verwendung für einen Herzschrittmacher vorgesehen. Es kann aber auch für andere Arten von Neuro- oder Muskelstimulationen verwendet werden. Ferner kann das Kabel auch dazu dienen, um von Körper-Zellen erzeugte, elektrische Signale einer Messvorrichtung zuzuführen. Das Kabel kann also einen elektrischen Strom von einer Stromquelle zu einem in einem Körperteil angeordneten Aktivator oder von einem in einem Körperteil angeordneten Sensor zu einer Messvorrichtung leiten.

Die Fasern sind im Querschnitt, abgesehen von rauhigkeitsbedingten Unregelmässigkeiten, vorzugsweise kreisförmig, könnten aber möglicherweise eine andere, mehr oder weniger von einem Kreis abweichende, rundliche Querschnittsform haben. In einem solchen Fall soll unter der Dicke der Fasern deren maximale Querschnittsabmessung verstanden werden.

Die Erfindung beruht auf der Erkenntnis, dass beim praktischen Einsatz von Kabeln für Herzschrittmacher und ähnliche Einrichtungen Biegungen bis hinab zu Krümmungsradien von etwa 1 bis 2 mm auftreten, wobei der Durchmesser der dünnsten, gebräuchlichen und machbaren Kabel auch etwa im Bereich von 1 bis 2 mm liegt. Wenn die Dicke der Fasern erfindungsgemäss weniger als 20 µm und vorzugsweise höchstens 15 µm beträgt, können kritische Krümmungsradien von weniger als 2 mm, vorzugsweise weniger als 1,5 mm oder sogar nur ungefähr 1 mm oder noch weniger erzielt werden. Im übrigen kann die Dicke der Fasern zum Beispiel mindestens oder mehr als 5 µm betragen.

Das Kabel kann nur ein einziges oder vorzugsweise zwei oder mehr Bündel von Fasern aufweisen. Das bzw. jedes Bündel kann je nach der Anzahl vorhandener Bündel und dem gewünschten elektrischen Leitwert, d.h. Reziprokwert des elektrischen Widerstandes mindestens 100, mindestens 200, mindestens 500 oder weniger, mindestens oder ungefähr 1000 Fasern und bis beispielsweise höchstens 3000 Fasern enthalten.

Die zum gleichen Bündel gehörenden Fasern können parallel zu dessen Längsrichtung und/oder Längsachse verlaufen oder um die letztere herumgedreht sein. Die zum gleichen Bündel gehörenden Fasern sollen vorzugsweise einerseits derart locker zusammengehalten sein, dass sich benachbarte Fasern begrenzt gegeneinander bewegen können, andererseits aber vorzugsweise doch zumindest stellenweise mit einer gewissen Mindestdruckkraft aneinander anliegen, die ausreichend ist, um trotz noch näher erörterter Oxydschichten zumindest stellenweise elektrische Verbindungen zwischen benachbarten Fasern zu ermöglichen.

Das bzw. jedes zu einem Kabel gehörende Bündel von Fasern kann zumindest bei den meisten seiner Abschnitte mit der Längsrichtung des Kabels einen Winkel bilden und nämlich zickzack- und/oder wellen- und/oder wendelförmig sein. Das Kabel kann zum Beispiel durch Flechten, Wirken, Weben oder Zusammendrehen lose und locker zusammengehaltene Bündel von Fasern aufweisen, sodass zum Beispiel zumindest stellenweise Zwischenräume zwischen benachbarten Bündeln vorhanden sind. Die Bündel können sich dann begrenzt gegeneinander bewegen, sodass ein aus der Gesamtheit der Fasern bestehenden Fasergebilde eine gute Deformierbarkeit aufweisen kann, die über die Dehnbarkeit einer einzelnen Faser heraus gehende Dehnungen des Fasergebildes sowie natürlich auch Biegungen und Torsionen von diesem ermöglicht und eine gewisse Formelastizität ergibt. Während die elastische Dehnbarkeit einer einzelnen metallischen Faser zum Beispiel typischerweise in der Grösse von 1% liegt, kann das Fasergebilde je nach Bedarf bis zu 10% oder sogar bis zu 20% dehnbar gemacht werden und hat im übrigen auch nur eine geringe Biege-und Torsionssteifigkeit. Für Anwendungen, bei denen keine besonders hohen Dehnbarkeit der Kabel erforderlich ist, kann man jedoch auch vorsehen, das bzw. jedes Faserbündel eines Kabels parallel zur Längsrichtung des letzteren anzuordnen.

Es ist also zur Erzielung einer guten Deformierbarkeit, insbesondere Biegbarkeit und Verdrehbarkeit des Kabels vorteilhaft, wenn die Fasern mindestens im allgemienen schief zur Längsrichtung des Kabels verlaufen. Dies kann gemäss den vorgängigen Erörterungen dadurch erreicht werden, dass die Fasern um die Längsachse des Bündels herum gedreht werden, zu dem sie gehören, und/oder dass das bzw. jedes Bündel derart angeordnet wird, dass es mindestens im allgemeinen schief zur Längsrichtung des Kabels verläuft.

Falls die einzelnen Fasern um die Längsachse des von ihnen gebildeten Bündels herumgedreht sind, sollen die dadurch gebildeten Wendeln sehr gestreckt und/oder langgezogen sein. Dabei ist mit gestreckt oder langgezogen gemeint, dass die Fasern derart grosse Steigungen haben sollen, dass die Länge oder, genauer gesagt, abgewickelte Länge jeder Faser nur verhältnismässig wenig, nämlich zweckmässigerweise höchstens 20%, vorzugsweise höchstens 10% und beispielsweise höchstens oder ungefähr 5% grösser ist als die Länge des Bündels, zu dem die betreffende Faser gehört. Wenn das bzw. jedes Bündel einen zickzack-, wellen- oder wendelförmigen Verlauf hat, soll die von dem bzw. jedem Bündel gebildete zickzack-, wellen- oder wendelförmige Linie ebenfalls sehr gestreckt und/oder langgezogen sein, so dass die Länge oder, genauer gesagt, abgewickelte Länge des Bündels sowie auch die abgewickelten Längen der einzelnen Fasern des Bündels nur verhältnismässig wenig grösser sind, als die Länge des ganzen Kabels. Vorteilhafterweise soll nämlich die Länge des bzw. jedes Bündels und vorzugsweise auch die Länge jeder seiner Fasern höchstens 50% und zum Beispiel höchstens oder ungefähr 30% grösser sein als die Länge des Kabels selbst. Wenn zum Beispiel ein Kabel eine Wendel aufweist, die aus einem Bündel oder aus mehreren in der Art eines

4

mehrgängigen Gewindes verlaufenden Bündeln gebildet ist, sollen zwischen den einander am nächsten benachbarten Bündelwindungen freie Zwischenräume vorhanden sein, die vorzugsweise wesentlich grösser sind als die Bündeldicke und beispielsweise ein Vielfaches von dieser betragen. Das Verhältnis zwischen der Länge eines Faserbündels oder einer einzelnen Faser und der Länge des Kabels ist gleich dem Reziprokwert vom Cosinus des Winkels, den das Bündel bzw. die Faser mit der Längsrichtung des Kabels bildet. Falls der Winkel entlang dem Kabel variiert, ist das Verhältnis durch einen gewichteten Mittelwert des Winkels gegeben. Die angegebenen Grenzwerte für die Längenunterschiede zwischen den Fasern, Bündeln und Kabeln können also eingehalten werden, indem man den Winkel, den die Faserbündel und ihre Fasern mit der Längsrichtung des Kabels bilden, höchstens etwa 30° oder sogar nur höchstens oder ungefähr 20° macht. Wenn die Fasern höchstens 50% oder sogar nur höchstens oder ungefähr 30% länger gemacht werden als das Kabel, kann erreicht werden, dass der elektrische Widerstand einer Faser höchstens um einen entsprechenden Prozentsatz grösser ist als der Widerstand einer parallel zur Kabellängsrichtung verlaufenden Faser. Der allenfalls nicht zur Kabellängsrichtung parallele Verlauf der Fasern und/oder Faserbündel bewirkt dann eine wesentlich kleinere Widerstandserhöhung als die wendelartige Formgebung der Drähte bei vielen vorbekannten Kabeln, bei denen benachbarte Wendelwindungen mindestens annähernd aneinander anliegen und die Länge eines Drahtes ein Vielfaches der Länge des Kabels beträgt. Da die Faserbündel und deren Fasern bei den erfindungsgemässen Kabeln ohne Verlust der Flexibilität derart angeordnet werden können, dass sie mit der Längsrichtung des Kabels höchstens relativ kleine Winkel bilden, kann mit einem erfindungsgemässen Kabel auch dann ein verhältnismässig kleiner, elektrischer Widerstand erzielt werden, wenn der spezifische, elektrische Widerstand des Fasermaterials nicht besonders niedrig ist und/oder wenn die totale Querschnittfläche der Fasern nur relativ klein ist.

Die Fasern weisen ein metallisches Material mit mindestens einem Metall auf und können bei einer bevorzugten Ausführungsform aus einer Legierung bestehen, die zusätzlich zu dem das Basismetall bildenden Titan mindestens eines der Metalle, Niob, Tantal, Zirkon, Chrom, Molybdän, Eisen und Aluminium enthält. Der Titan-Anteil an der Legierung soll gewichtsmässig am grössten sein und im allgemeinen mindestens 50 Gew.-% betragen. Ferner sollen die Fasern abgesehen von allenfalls vorhandenen Verunreinigungen vorteilhafterweise keine anderen Metalle als Titan und die vorgängig genannten Legierungsbestandteile enthalten. Die die Fasern bildende Legierung soll vorzugsweise kaltverformt sein, worunter zu verstehen ist, dass zumindest der Schlussteil bzw. die Schlussphase des zur Herstellung der Fasern dienenden, noch näher erläuterten Verformungsvorgangs eine Kaltverformung ist.

Das Kabel ist vorzugsweise zumindest aussen mit einer elektrischen Isolation versehen, die aus einem gummielastischen, gut biegbaren und biokompatiblen Material, beispielsweise einem Elastomer auf Polyurethan- oder Siliconbasis besteht. Für die Verwendung für einen Herzschrittmacher und auch für andere Zwecke ist es vorteilhaft, wenn das Kabel schlauchförmig ausgebildet ist und einen am einen Ende offenen und am anderen Ende geschlossenen Hohlraum besitzt, in den vorübergehend ein sogenanntes Stilett eingesetzt werden kann. Die Fasern eines Kabels können zusammen einen einzigen Leiter bilden oder in gegeneinander isolierte Gruppen unterteilt sein, von denen jede einen elektrischen Leiter bildet.

Titan und die genannten Titanlegierungen haben auch eine gute Festigkeit und einen verhältnismässig niedrigen Elastizitätsmodul, wobei die letztgenannte Eigenschaft besonders vorteilhaft ist, weil sie beim Biegen die Beanspruchung klein hält. Titan gehört zu den Metallen des sogenannten alpha-Typs. Titanlegierungen können abhängig von ihrer Zusammensetzung verschiedene Phasenstrukturen haben und je nach dem zum alpha-, alpha-beta- oder beta-Typ gehören. Die TiNbTaAl-Legierung mit 3 Gew.-% Niob, 1 Gew.-% Tantal, 6 Gew.-% Aluminium und dem Rest Titan gehört zum Beispiel zum alpha-Typ. Zu den Legierungen des alpha-beta-Typs gehören zum Beispiel die TiAlFe-Legierung mit 5 Gew.-% Al, 2,5 Gew.-% Eisen und dem Rest Titan sowie die TiNbAl-Legierung mit 7 Gew.-% Niob, 6 Gew.-% Aluminium und dem Rest Titan. Zu den Legierungen des beta-Typs gehören die TiNb-Legierung mit 40 Gew.-% Niob und dem Rest Titan sowie die TiMoZrAl-Legierung mit 15 Gew.-% Molybdän, 5 Gew.-% Zirkon, 3 Gew.-% Aluminium und dem Rest Titan. Der Elastizitätsmodul liegt typischerweise für Titan sowie die Legierungen des alpha-Typs und des alpha-beta-Typs bei 100 bis 120 GPa und für die Legierungen des beta-Typs bei ungefähr 65 bis 110 GPa, wobei die Werte von der Wärmebehandlung abhängig sind. Die Legierungen des beta-Typs haben eine kubische Struktur und, wie auch in gewissem Ausmass diejenigen des alpha-beta-Typs, eine grössere plastische Verformbarkeit als Titan und die Legierungen des eine hexagonale Struktur aufweisenden alpha-Typs, sodass Legierungen des alpha-beta- und vor allem des beta-Typs vorteilhafter sind als Materialien des alpha-Typs. Zudem kann die Festigkeit bei den Legierungen des beta-Typs durch eine Wärmebehandlung, wie Lösungsglühen und/oder Auslagern, erhöht werden.

Die erwähnte, kaltverformte TiNb-Legierung des beta-Typs hat zum Beispiel einen Elastizitätsmodul von ungefähr 69 oder 70 GPa. Die der üblichen Zugfestigkeit entsprechende Bruchspannung, d.h. die Spannung, bei der bei einmaliger Zugbelastung ein Bruch stattfindet, beträgt 0,88 GPa, während die Ermüdungsspannung, d.h. die für beliebig oft wiederholte Biegungen gerade noch zulässige Spannung, etwa 0,4 GPa beträgt. Falls man die Formel (2) nach dem Faserdurchmesser d auflöst, für E und für $\sigma_z$ oder, genauer gesagt, eigentlich anstelle von diesem den Elastizitätsmodul bzw. die Ermüdungsspannung von TiNb einsetzt und einen kritischen Krümmungsradius von 1,5 mm vorgibt, ergibt sich ein Faserdurchmesser d von 17 μm.

Wenn man mit der bereits erwähnten, für vorbekannte Kabel benutzten PtIr-Legierung mit E = 150 GPa und einer Ermüdungsspannung 0,3 GPa einen kritischen Krümmungsradius von 1,5 mm erreichen möchte, müsste der Faserdurchmesser etwa 6 μm betragen und also beträchtlich dünner gemacht werden als bei der

EP 0 312 495 A2

TiNb-Legierung. Bei einer hochfesten Kobaltlegierung mit E = 230 GPa und einer Ermüdungsspannung von 0,8 GPa müsste der Faserdurchmesser 10 µm gemacht werden, wobei Platin-Iridium-und vor allem Kobalt-Legierungen wesentlich schlechter plastisch verformbar sind als Titan und Titan-Legierungen, sodass die Herstellung ausreichend biegbarer Fasern aus Platin- Iridium- und Kobalt- Legierungen relativ schwierig wäre.

Die Biegesteifigkeit S einer einzelnen Faser ist gleich dem Produkt E I, wobei E und I die in der Formel (1) angegebenen Bedeutungen haben. Für ein Bündel lose zusammengehaltener, paralleler oder eventuell leicht verdrillter Fasern ist die Biegesteifigkeit zumindest nährungsweise gleich dem Produkt aus der Anzahl Fasern mal die Biegesteifigkeit einer einzelnen Faser. Die Biegesteifigkeit eines Bündels von Fasern ist wesentlich geringer, als diejenige eines einzelnen Drahtes, dessen Querschnittsfläche gleich der gesamten Querschnittsfläche der Fasern eines Faserbündels ist. Ein Bündel mit 200 Fasern, die aus der genannten TiNb-Legierung bestehen und einen Durchmesser von 17 µm haben, hat zum Beispiel eine Biegesteifigkeit von $5{,}7 \cdot 10^{-8}$ $Nm^2$. Ein einzelner Draht aus dem gleichen Material und mit der Querschnittsfläche von 200 Fasern hält einen Durchmesser von 240 µm sowie eine etwa $1{,}1 \cdot 10^{-5}$ $Nm^2$ betragende und also 200 mal grössere Biegefestigkeit.

Der spezifische, elektrische Widerstand von Titan beträgt 42 µΩ cm und derjenige der genannten Titan-Legierungen etwa 50 bis 90 µΩ cm. Zum Vergleich sei angegeben, dass der spezifische Widerstand der bei vorbekannten Kabeln verwendeten PtIr-Legierung mit 10 Gew-% Iridium und dem Rest Platin 25 µΩ cm beträgt und derjenige von für bekannte Kabel verwendeten Kobalt-Legierungen in der Grösse von 70 µΩ cm liegt. Titan und seine Legierungen haben also zwar einen grösseren spezifischen Widerstand als eine PtIr-Legierung, was jedoch bei erfindungsgemässen Kabeln dadurch kompensiert werden kann, dass die Länge der Fasern, wie bereits erwähnt, höchstens verhältnismässig wenig länger zu sein braucht als die Länge des Kabels, während bei den vorbekannten Kabeln die Drahtlänge üblicherweise ein Vielfaches der Kabellänge beträgt.

Titan ist reaktiv in Bezug auf Sauerstoff. Wenn sich nämlich zum Beispiel eine aus einer Titan-Legierung bestehende Faser in einer freien oder gebundenen Sauerstoff enthaltenden, oxydierend wirkenden Umgebung befindet und etwa der Einwirkung von Luftsauerstoff oder elektrolytischen Flüssigkeiten ausgesetzt ist, bildet sich an der Faseroberfläche eine filmartige, kompakte Metalloxydschicht, nämlich beim Titan $TiO_2$. Auch die als mögliche Legierungsbestandteile erwähnten Metalle Niob, Tantal, Zirkon, Chrom und Aluminium sind reaktiv in Bezug auf Sauerstoff und Eisen wird zumindest als Legierungsbestandteil einer Titanbasis-Legierung ebenfalls von einer Oxydschicht bedeckt.

Die auf der Oberfläche jeder Faser durch spontane Oxydation entstehende Oxydschicht hat typischerweise eine in der Grösse von 3 nm liegende Schichtdicke. Falls dickere Oxydschichten erwünscht sind, können deren Dicken durch anodische Oxydation bis auf etwa 50 nm oder mehr erhöht werden.

Die spontan entstandenen und eventuell zusätzlich durch anodische Oxydation verstärkte Oxydschichten schützen das von ihnen bedeckte Metall gegen Korrosion. Wenn daher die üblicherweise vorhandene Isolation eines Kabels beschädigt und dadurch Fasern zum Beispiel in Kontakt mit Blut oder einer anderen Körperflüssigkeit oder festen Körperzellen gelangen sollten, findet trotzdem zumindest praktisch keine Korrosion statt. Im übrigen sind Titan und die genannten Titan-Legierungen auch nicht toxisch und zeigen also ein biologisch träges Verhalten.

Eine durch spontane Oxydation auf der Oberfläche einer Faser entstandene und möglicherweise zusätzlich durch anodische Oxydation verstärkte Oxydschicht ist, wenn keine nennenswerten mechanischen Druckkräfte auf sie ausgeübt werden, elektrisch isolierend, wobei die Isolierwirkung von der Polarität der Spannung abhängig ist. Bei anodischer Polarisation, d.h. wenn am Metall bezüglich dessen Umgebung eine positive Spannung anliegt, ist die Oxydschicht bis zu einer vom Metall abhängigen, mindestens 3 V betragenden Durchbruchspannung isoliert. Bei Spannungen mit umgekehrter Polarität fliessen bis mindestens 10 V höchstens vernachlässigbar kleine Ströme durch die Oxydschicht. Wenn aber aus Titan oder einem der genannten Titanbasis-Legierungen bestehende Drähte eines isolierten Kabels wegen eines Defekts der Isolation in Berührung mit Körperflüssigkeit oder -gewebe gelangen sollte, verhindert die erwähnte Oxydschicht bei der defekten Stelle, zumindest wenn keine Druckkräfte auf die Oxydschichten ausgeübt werden, eine unerwünschte, elektrisch leitende Verbindung mit dem Körper. Falls hingegen Druckkräfte auf die Fasern und deren Oxydschichten ausgeübt werden, verlieren die letzteren ihre elektrisch isolierende Wirkung. Zwischen benachbarten Fasern, die einander zumindest stellenweise mit einer gewissen Mindest-Druckkraft berühren, können bei den Berührungsstellen daher trotz der Oxydschicht elektrisch leitende Verbindungen entstehen. Falls daher in einen Bündel von Fasern eine der letzteren brechen sollte, können benachbarte Fasern die Bruchstelle elektrisch überbrücken, sodass der Bruch keine nennenswerte Erhöhung des elektrischen Widerstandes bewirkt. Im übrigen können die zu einem oder mehreren Faserbündel gehörenden Fasern bei den Enden des Kabels selbstverständlich ohne weiteres durch Festklemmen elektrisch leitend miteinander und mit einem elektrisch leitenden Körper, wie einer Kontakt-Elektrode oder einen Anschluss eines elektronischen Impulsgebers verbunden werden.

Wie bereits erwähnt, werden die Kabel im allgemeinen mit einer gummielastischen, elektrischen Isolation versehen. Da die auf den Oberflächen von Fasern aus Titan oder den genannten Titan-Legierungen vorhandenen Oxydschichten die Fasern gegen Korrosion schützen und elektrisch isolierend wirken, kann die gummielastische Isolation verhältnismässig dünn bemessen werden. Dies trägt zur Erzielung kleiner Querschnittsabmessungen, zum Beispiel kleiner Durchmesser, und kleiner Steifigkeiten der Kabel bei.

6

Metallische Fasern mit Durchmessern von weniger als 20 µm sind kaum noch mit zur Herstellung einzelner Drähte gebräuchlichen Verfahren herstellbar. Solche Fasern lassen sich jedoch bündelweise herstellen, indem vorzugsweise einen kreisförmigen Querschnitt aufweisende, verhältnismässig dicke, aus dem Material der zu bildenden Fasern bestehende Drähte, deren Anzahl mit der gewünschten Anzahl Fasern eines Bündels übereinstimmt, in Bohrungen eines Blocks eingesetzt werden, der aus einem anderen metallischen Material besteht als die Fasern. Das Material des eine Matrix bildenden Blocks soll vorzugsweise weicher sein als das Fasermaterial und in Bezug auf Verlängerungs-Verformungen mindestens eine ähnliche Duktilität wie das letztere haben. Die Matrix kann zum Beispiel aus Kupfer oder einer Kupfer-Nickel-Legierung bestehen. Der aus einer Matrix und den darin eingebetteten Drähten bestehende Verbund-Rohling kann durch warmes und/oder kaltes Verformen, wie Pressen und/oder Walzen und/oder Ziehen, stufenweise gestreckt und im Durchmesser reduziert werden, so dass die Dicke der Drähte auf die gewünschte Dicke der Fasern abnimmt. Vorzugsweise ist zumindest der Schlussteil bzw. die Schlussphase des Verformungsvorgangs eine Kaltverformung. Die Verformung kann zum Beispiel durch Warmpressen und anschliessendes Kaltziehen erfolgen. Bei einem solchen eine Querschnittsflächenverminderung sowie Verlängerung des Rohlings und der Drähte bewirkenden Verformungsvorgang bleibt das Volumen des Rohlings sowie der einzelnen Drähte erhalten. Ein ähnliches Verformungsverfahren ist, wie bereits erwähnt für die Herstellung von Supraleitern mit in eine Kupfermatrix eingebetteten Fasern aus einer Titan-Niob-Legierung bekannt. Während bei den fertigen Supraleitern die Matrix erhalten bleibt, wird diese für die Bildung von Fasern für ein Kabel nach der Verformung mit einer das Fasermaterial nicht angreifenden Säure, zum Beispiel Salpetersäure herausgelöst.

Wie bereits erläutert, kann das bzw. jedes Faserbündel des fertiggestellten Kabels einen zickzack-, wellen- und/oder wendelförmigen Verlauf haben, wobei zum Beispiel mehrere Bündel ein Geflecht, Gewirke, Gewebe und/oder einen Schlauch und/oder ein Seil bzw. einen Zwirn bilden. Ein solcher Verlauf des bzw. jedes Faserbündels kann durch einen Formgebungsvorgang erzielt werden, bei dem die Fasern zumindest im wesentlichen nur noch gebogen werden, d.h. im Gegensatz zur vorher bei ihrer Herstellung aus Drähten stattgefundenen Verformung nicht mehr oder zumindest nicht mehr wesentlich dünner und länger gemacht werden. Das bzw. jedes Faserbündel kann bei der genannten, ohne oder zumindest ohne wesentliche Querschnittsflächenverminderung sowie ohne Verlängerung der Fasern stattfindenden Formgebung bereits vor dem Herauslösen der Matrix zusammen mit dieser oder nach dem Herauslösevorgang verdrillt und/oder entsprechend der gewünschten Art des herzustellenden Kabels, als einzelnes Bündel zickzack-, wellen-oder wendelförmig geformt und/oder mit anderen Faserbündeln verbunden werden.

Wenn die Verdrillung und/oder Verflechtung und/oder sonstige Formgebung der Faserbündel vor dem Herauslösen der Matrix erfolgt, gewährleistet die feste Verbindung zwischen den Fasern und der Matrix, dass die Fasern ohne zu brechen gleich wie die sie umgebende Matrix gebogen werden. Die Duktilität und/oder Festigkeit der Fasern kann nach deren durch eine volumenerhaltende, verlängernde Querschnittsflächenverminderung von Drähten erfolgten Herstellung vor und/oder während und/oder nach dem Herauslösen der Matrix sowie vor und/oder während und/oder nach ihrer in der beschriebenen Weise ohne wesentliche Verlängerung und Querschnittsflächenveränderung stattfindenden Formgebung der Fasern auch noch durch eine Wärmebehandlung, wie Glühen, etwa Weichglühen oder Lösungsglühen, und/oder Auslagern, in erwünschter Weise beeinflusst werden. Falls die Fasern aus einer Titan-Legierung des beta-Typs bestehen und insbesondere falls zudem bei der zu ihrer Herstellung durch Verlängern und Querschnittsverminderung dienenden, mehrstufigen Verformung zumindest am Ende eine Kaltverformung stattfindet, kann die Festigkeit der Fasern noch durch eine Wärmebehandlung, wie Lösungsglühen und/oder Auslagern, erhöht werden. Ferner kann man die Fasern sofort nach der Herstellung eines Bündels von Fasern oder erst nach dessen zickzack-, wellen- oder wendelförmiger Verformung und/oder Verbindung mit anderen Faserbündeln, aber selbstverständlich erst nach dem Herauslösen der Matrix anodisch oxydieren. Dazu kann mindestens ein Faserbündel oder ein mehrere solche aufweisende Fasergebilde in einer oxydierenden, elektrolytischen Flüssigkeit, zum Beispiel verdünnter Phosphorsäure, bei im Behandlungsverlauf bis zu 50 V oder bis zu 100 V steigenden Spannungen oxydiert werden.

Der Erfindungsgegenstand soll nun anhand in der Zeichnung dargestellter Ausführungsbeispiele von Kabeln erläutert werden. In der Zeichnung zeigen

die Figur 1 eine schematisierte Schrägansicht eines Stücks eines Kabels mit miteinander zu einem schlauchförmigen Fasergebilde verflochtenen Bündeln von Fasern,

die Figur 2 eine schematisierte Seitanansicht eines längeren Stücks des Fasergebildes des in der Figur 1 dargestellten Kabels, ohne die Isolation,

die Figur 3 eine schematisierte Schrägansicht eines Stücks eines Kabels mit zwei Gruppen von elektrisch gegeneinander isolierten Bündeln von Fasern,

die Figur 4 eine schematisierte Schrägansicht eines Stücks eines Kabels mit drei elektrisch gegeneinander isolierten Bündeln von Fasern und

die Figur 5 eine schematisierte Schrägansicht eines Stücks eines Kabels mit nur einem Bündel von Fasern.

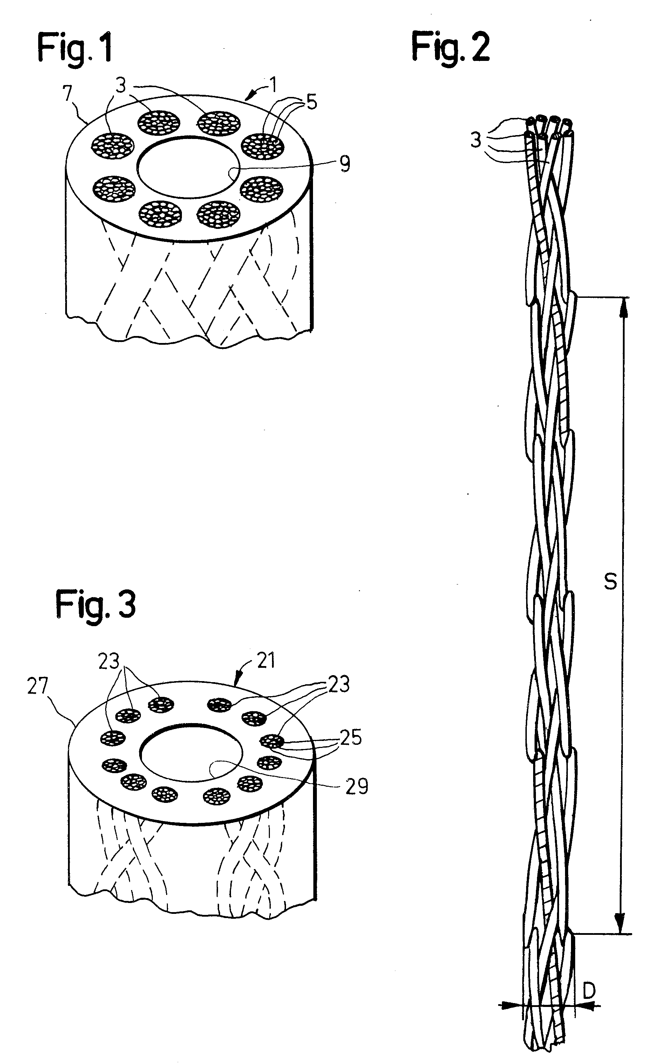

Das in der Figur 1 dargestellte, schlauchförmige Kabel 1 hat ein kreisringförmiges Profil und weist mehrere, nämlich mindestens vier und beispielsweise acht Bündel 3 auf, die beispielsweise eine ungefähr rund Querschnittsform haben und je eine Anzahl metallischer Fasern 5 enthalten, die ihrerseits einen kreisförmigen Querschnitt haben. Es sei bemerkt, dass in der Figur 1 nur ein Teil der tatsächlich vorhandenen Fasern gezeichnet worden ist, wobei die Durchmesser der Fasern und vor allem die Abstände der Fasern 5 im Vergleich zum Aussendurchmesser des Kabels 1 in überproportionaler Grösse gezeichnet wurden. Die Bündel 3 sind in eine elektrisch isolierende Isolation 7 mit einer im Querschnitt kreisförmigen Aussenfläche eingebettet. Die Isolation 7 enthält eine koaxiale, im Querschnitt ebenfalls kreisrunde Längsöffnung 9 und isoliert die Bündel 3 sowohl gegen aussen als auch gegen die Längsöffnung 9. Die Isolation 7 besteht aus einem gummielastischen, insbesondere gut biegbaren und biokompatiblen Material, beispielsweise einem Elastomer auf Polyurethan- oder Siliconbasis.

Die acht Bündel 3 mit ihren Fasern 5 sind elektrisch parallel geschaltet und bilden zusammen den elektrischen Leiter des Kabels 1. Die einzelnen Bündel 3 können gemäss der Figur 2 zu einem zusammenhängenden Fasergebilde verbunden, nämlich zu einem schlauchförmigen Geflecht verflochten sein, wobei die eine Hälfte der Bündel in der einen und die andere Hälfte der Bündel in der umgekehrten Drehrichtung um die Längsachse des Kabels herum verläuft. Der Flechtwinkel, d.h. der Winkel, den die Bündel 3 und auch jede ihrer Fasern mit der Längsachse des Kabels bilden, soll höchstens 50° und vorzugsweise wesentlich weniger betragen. Man kann jedem um die Längsachse des Kabels herumgedrehten Bündel 3 analog zur Steigung einer Schraubenlinie eine Steigung S zuordnen, die mindestens gleich dem fünffachen und vorzugsweise mindestens gleich dem zehnfachen Aussendurchmesser D des von den Bündeln 3 gebildeten, schlauchförmigen Geflechts ist. Damit die Bedeutung und Messung der Steigung S in der Figur 2 klarer ersehbar ist, wurde in der Figur 2 eines der Bündel durch eine Schraffur herausgehoben. Da die nur in der Figur 1 gezeichnete Isolation 7 die Bündel 3 beim Aussen-Umfang des Kabels 1 umschliesst, ist der Aussen-Durchmesser des Kabels 1 etwas grösser als der Durchmesser D. Zweckmässigerweise ist jedoch die Steigung S auch mindestens gleich dem fünffachen und vorzugsweise mindestens gleich dem zehnfachen Aussendurchmesser des Kabels 1. Die zu ein und demselben Bündel 3 gehörenden Fasern 5 können parallel zur Längsachse des betreffenden Bündel 3 verlaufen oder um die Bündel-Längsachse herumgedreht sein. Falls die Fasern 5 derart gedreht, d.h. miteinander verdrillt sind, ist die Steigung, mit der sie um die Längsachse des betreffenden Bündels herum verlaufen, zweckmässigerweise mindestens gleich dem fünffachen und vorzugsweise mindestens gleich dem zehnfachen Durchmesser D oder sogar zweckmässigerweise mindestens gleich dem fünffachen bzw. vorzugsweise mindestens gleich dem zehnfachen Aussendurchmesser des Kabels 1.

Die verschiedenen Bündel 3 sind vorteilhafterweise nur verhältnismässig lose und locker miteinander verflochten, sodass zwischen den sich am nächsten benachbarten Bündeln 3 mindestens stellenweise Zwischenräume vorhanden sind. Wie in der Figur 1 angedeutet, kann die Isolation 7 die zwischen den Bündeln 3 vorhandenen Zwischenräume mindestens stellenweise durchdringen. Die einander benachbarten Bündel können sich jedoch auch mindestens stellenweise berühren.

Wenn das Kabel 1 bei seiner Verwendung gedreht, gekrümmt oder tordiert wird, können sich die Bündel 3 bezüglich einander bewegen. Das Geflecht wird dadurch relativ stark dehnbar sowie auch gut bieg- und tordierbar, wobei bei den Deformationen auch die gummielastische Isolation 7 deformiert wird.

Jedes Bündel 3 kann zum Beispiel 100 Fasern 5 mit einem abgesehen von den rauhigkeitsbedingten Abweichungen kreisförmigen Querschnitt und einem 12 bis 13 μm betragenden Durchmesser aufweisen. Die gesamte Querschnittsfläche der zum gleichen Bündel 3 gehörenden Fasern 5 beträgt dann 0,012 mm². Ein Einzeldraht mit dieser Querschnittsfläche hätte einen Durchmesser von 0,125 mm. Da die Fasern 5 die von einem Bündel 3 eingenommene Querschnittsfläche ja nicht vollständig ausfüllen, ist diese etwas grösser als diejenige des genannten Einzeldrahtes und beträgt ungefähr 0,02 mm², was einem Bündeldurchmesser von etwa 0,16 mm entspricht. Dies erlaubt, Kabel mit relativ kleinem Durchmesser herzustellen, wobei der Durchmesser D des schlauchförmigen Geflechts beispielsweise in der Grösse von 0,6 bis 1,5 mm liegen kann. Der Aussendurchmesser des gesamten Kabels kann dann abhängig von der Dicke der Isolation 7 noch einige, wenige Zehntelmillimeter grösser sein.

Die Fasern 5 können aus einer TiNb-Legierung mit 40 Gew.-% Niob und dem Rest Titan bestehen, die nach der Bildung der Fasern durch Verformen eventuell noch einer Wärmebehandlung unterzogen wurde und einen Elastizitätsmodul von 70 GPa und eine Ermüdungsspannung von etwa 0,4 GPa hat. Wenn man diese beiden Werte in die Formel (2) einsetzt, ergibt sich ein kritischer Krümmungsradius von nur 0,9 mm. Die TiNb-Legierung hat bei einmaliger Zugbeanspruchung eine Bruchspannung von etwa 0,8 GPa. Ein Bündel 3 mit 100 Fasern 5 hat daher eine Zugfestigkeit von etwa 10 N. Die Zugfestigkeit eines aus mehreren, zum Beispiel gemäss der Figur 1 aus acht, miteinander verflochtenen Bündeln bestehenden Schlauchs ist zu mindest ungefähr gleich dem Produkt Anzahl Bündel mal Zugfestigkeit eines Bündels.

Die TiNb-Legierung hat einen spezifischen, elektrischen Widerstand von 86 μΩ cm. Ein Bündel 3 mit 100 parallel zu seiner Längsrichtung verlaufenden, den genannten Durchmesser aufweisenden Fasern 5 hat einen Widerstand von 70 Ω pro Meter Länge. Wenn der Flechtwinkel, d.h. der Winkel zwischen der Kabellängsrichtung und den Bündeln 30° beträgt, ist die Länge eines Bündels 3 und jeder Faser 5 gleich der 1,16-fachen Länge des Kabels. Dessen acht elektrisch parallel geschaltete Bündel ergeben dann einen Widerstand von ungefähr 10,1 Ω pro Meter Länge des Kabels.

Die Anzahl der zum Kabel gehörenden und miteinander verflochtenen Faserbündel kann selbstverständlich variiert und zum Beispiel ohne weiteres auf 10 erhöht werden, wobei im letzeren Fall die Zugfestigkeit des schlauchförmigen Geflechts auf ungefähr 100 N erhöht und der elektrische Widerstand des Kabels auf ungefähr 8,1 Ω pro Meter Kabellänge reduziert wird. Selbstverständlich kann auch die Anzahl der pro Bündel vorhandenen Fasern verändert auch insbesondere erhöht werden. Ein Bündel kann zum Beispiel ohne weiteres ungefähr 200 oder ungefähr 500 oder noch mehr Fasern enthalten.

Bei einem anderen Ausführungsbeispiel enthält jedes in der Figur 1 und 2 dargestellte Faserbündel 100 Fasern mit kreisförmigem Querschnitt und einem Durchmesser von 17 μm. Die Fasern 5 können zum Beispiel statt aus der der genannten TiNb-Legierung aus einer TiMoZrAl-Legierung mit 15 Gew.-% Molybdän, 5 Gew.-% Zirkon, 3 Gew.-% Aluminium und dem Rest Titan bestehen, wobei die Legierung nach der Herstellung der Fasern noch einer Auslagerung unterzogen wird. Dieses Fasermaterial hat einen Elastizitätsmodul von 90 GPa und eine zulässige Ermüdungsspannung von etwa 0,7 GPa, sodass die Formel (2) einen kritischen Krümmungs radius von 1,2 mm ergibt. Das Fasermaterial hat bei einmaliger Zugbeanspruchung eine Bruchspannung von 1,2 GPa, sodass ein Bündel mit 100 Fasern 5 eine Zugfestigkeit von etwa 30 N hat. Der spezifische Widerstand der TiMoZrAl-Legierung beträgt 90 μΩ cm, sodass ein Faserbündel einen Widerstand von 35 Ω pro Meter Länge hat.

Wenn das Kabel 1 für die Verwendung als Teil eines Herzschrittmachers bestimmt ist, kann am herzseitigen Ende des Kabels als Aktivator eine Kontakt-Elektrode angeordnet sein, die den von der Gesamtheit der Fasern 5 gebildeten Leiter mit dem zu stimulierenden Gewebe verbindet. Die Kontakt-Elektrode ist dabei vorzugsweise mit einem die Längsöffnung 9 dicht abschliessenden Abschlussteil, beispielsweise einer den Endabschnitt der Isolation 7 umschliessenden und an dieser befestigten Hülse, versehen. Am andern Ende kann das Kabel mit einem Anschluss versehen werden, der an einen elektrischen Impulsgeber anschliessbar ist. Die Kontakt-Elektrode und der Anschluss können lösbar oder unlösbar am Kabel befestigt werden und bilden dann mit diesem zusammen eine elektrische Leitvorrichtung. Bei eingesetztem Herzschrittmacher verbindet das Kabel 1 den Impulsgeber elektrisch leitend mit der Kontakt-Elektrode. Das Kabel 1 ist im übrigen derart ausgebildet, dass von seinem an den Impulsgeber anschliessbaren Ende her vorübergehend ein sogenanntes Stilett, das durch einen drahtförmigen Dorn gebildet ist oder einen solchen aufweist, in die Längsöffnung 9 eingeschoben werden kann. Das mit einer Kontakt-Elektrode versehene Ende des Kabels kann dann unter Verwendung dieses Stiletts durch eine Ader hindurch in das Herz des Patienten hinein geschoben werden. Wenn man danach das Stilett aus der Längsöffnung 9 herauszieht, kann die letztere eventuell auch beim zum Anschliessen an den Impulsgeber bestimmten Ende des Kabels 1 durch einen Abschlussteil abgeschlossen werden.

Das in der Figur 3 dargestellte, zweiadrige Kabel 21 weist zwei Gruppen von in eine Isolation 27 eingebetteten Bündeln 23 mit je einer Anzahl Fasern 25 auf, wobei im Innern des Kabels eine freie Längsöffnung 29 vorhanden ist. Die zur gleichen Gruppe gehörenden Bündel 23 sind miteinander verflochten und bilden elektrisch gegeneinander isolierte, zum Führen verschiedener, elektrischer Spannungen dienende Leiter. Jede der beiden Bündel-Gruppen kann etwa 4 bis 8 und zum Beispiel gemäss der Figur 3 etwa 6 Bündel 23 enthalten, von denen jedes 100 Fasern oder mehr enthält. Die beiden Bündel 23 verlaufen parallel zur Längsachse des Kabels 21. Der Flechtwinkel kann wie beim in der Figur 1 und 2 dargestellten Ausführungsbeispiel in der Grösse von 30° liegen. Da jedoch die Bündel 23 nicht vollständig um die Längsachse des Kabels herum verlaufen, sind sie nicht wendel-, sondern eher zickzack- oder wellenförmig und können daher stellenweise, nämlich bei den Scheiteln bzw. Wellenbergen kurze, mehr oder weniger parallel zur Längsachse des Kabels verlaufenden Abschnitte haben. Im übrigen könnten die beiden Bündel-Gruppen statt parallel zur Kabel-Längsachse noch als Ganzes wendelartig um diese herumgedreht werden. Die beiden Bündel 23 können beim einen Ende des Kabels 21 beispielsweise elektrisch mit zwei separaten Kontakt-Elektroden und beim andern Ende mit zwei separaten Anschlüssen verbunden sein.

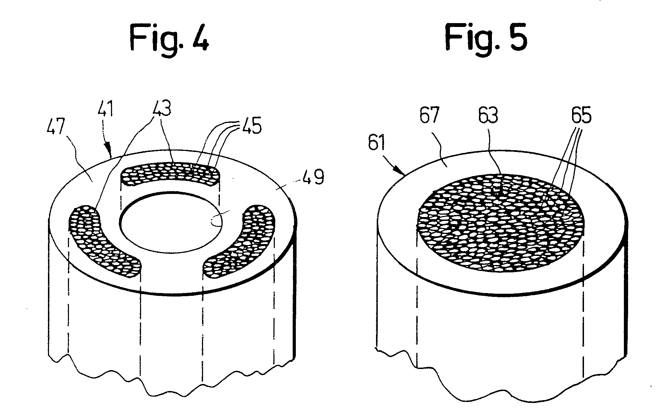

Das in der Figur 4 dargestellte, dreiadrige Kabel 41 weist drei im Querschnitt bogenförmige Bündel 43 mit Fasern 45 auf, die beispielsweise leicht verdrillt oder eventuell parallel zur Längsrichtung des betreffenden Bündels 43 sind. Die Bündel 43 sind in dem Mantel einer schlauchförmigen Isolation 47 mit kreisringförmigem Querschnitt eingebettet und durch diese Isolation gegen aussen, gegen die zentrale Längsöffnung 49 und gegeneinander isoliert. Die Bündel 43 verlaufen parallel zur Längsachse des Kabels, könnten aber auch um diese herumgewunden sein. Da das Kabel 41 drei elektrische, gegeneinander isolierte, je nur aus einem einzigen Faserbündel bestehende Leiter aufweist, weisen die Bündel zweckmässigerweise mindestens etwa 500 oder mindestens ungefähr 1000 Fasern auf.

Das in der Figur 5 dargestellte Kabel 61 bildet im Querschnitt einen vollen Kreis und weist einen zentral angeordneten, elektrischen Leiter auf, der aus einem parallel und koaxial zur Längsachse des Kabels verlaufenden Bündel 63 mit beispielsweise mindestens 500 oder mindestens 1000 Fasern 65 besteht, die beispielsweise verdrillt, d.h. um die Kabel-Längsachse herumgedreht sind, oder möglicherweise parallel zu dieser verlaufen. Der Leiter ist von einer im Querschnitt kreisförmigen Isolation 67 umschlossen und könnte im übrigen statt nur aus einem einzigen Faserbündel aus mehreren solchen bestehen, die um die Kabellängsachse herumgedreht, d.h. miteinander verdrillt, oder miteinander verflochten sind.

Die metallischen Fasern der in den Figuren 3, 4 und 5 dargestellten Kabel können zum Beispiel aus einer der für die Fasern 3 angegebenen Legierungen bestehen und auch gleiche oder ähnliche Querschnittsabmessungen haben wie die Fasern 3.

Die für die Bildung der in den verschiedenen Figuren dargestellte Kabel benötigten Fasern können gemäss dem in der Einleitung beschriebenen Herstellungsverfahren und dessen Varianten hergestellt werden. Bei diesem Herstellungsverfahren können zum Beispiel jeweils durch Verformen und Herauslösen des Matrix-Metalls Faserbündel hergestellt werden, von denen jedes gerade die für ein Faserbündel eines Kabels vorgesehene Anzahl Fasern enthält. Dies erlaubt, die Faserbündel für die Herstellung von Kabeln gemäss den Figuren 1 oder 3 zu verflechten, bevor das Matrix-Metall mit einer Säure herausgelöst wird. Man kann jedoch auch Faserbündel herstellen, die eine kleinere oder grössere Anzahl von Fasern enthalten, als sie für ein Bündel eines Kabels vorgesehen ist, und dann je nach dem mehrere der hergestellten Bündel zu einem Bündel für das Kabel zusammenlegen oder eines der hergestellten Faserbündel in mehrere zur Bildung von Kabeln bestimmte Bündel aufteilen.

Die Kabel können noch in anderer Weise modifiziert werden. Beispielsweise könnte man im Querschnitt ungefähr elliptische oder eventuell sogar rechteckige Faserbündel vorsehen. Ferner könnte man mehrere zusammen einen Leiter bildende Faserbündel ohne Verflechtung in der Art eines mehrgängigen Schraubengewindes um die Längsachse des Kabels herumdrehen oder parallel zur Kabel-Längsachse verlaufen lassen. Ferner könnte man bei einem nur einen einzigen Leiter bildenden Kabel die Fasern im Querschnitt gleichmässig über eine Ringfläche verteilen. Die in den Figuren 4 und 5 dargestellten Kabel könnten dahingehend geändert werden, dass jeder bzw. der Leiter in mehrere Faser bündel unterteilt wird, die miteinander verflochten sind, wobei die Faserbündel im Fall der Figur 5 statt miteinander verflochten miteinander verdrillt, d.h. um Kabel-Längsachse herumgedreht sein könnten.

Ferner wäre es möglich, ein Kabel mit zwei je aus mindestens einem Bündel von Fasern bestehenden Leitern vorzusehen, bei dem der eine Leiter den anderen koaxial umschliesst, wobei die beiden Leiter im Querschnitt durch eine ringförmige Schicht der Isolation getrennt wären.

Statt bei den eine zentrale Längsöffnung besitzenden Kabeln erst vor dem Einführen der Kabel in einen menschlichen oder tierischen Körper vorübergehend ein Stilett in die Längsöffnung der Kabel einzuschieben, könnte man die Kabel bereits bei ihrer Herstellung mit einem in ihrem Innern angeordneten, zur Bildung eines Stiletts dienenden Stahldraht versehen. Bei der Verwendung eines Kabels müsste man in diesem Fall natürlich kein Stilett mehr in das Kabel einschieben, sondern lediglich nach der Einführung des Kabels in einen Körper noch das Stilett herausziehen.

Ferner müsste man die Fasern bei den schlauchförmigen Kabeln auch nicht unbedingt direkt in die Isolation einbetten, sondern könnte eventuell nur einen den oder die Leiter aussen umschliessenden, isolierenden Mantel und eventuell noch einem dazu koaxialen, sich innerhalb des Leiters oder der Leiter befindenden Schlauch aus Isolationsmaterial vorsehen.

### Patentansprüche

1. Elektrisches Kabel für die Durchführung mindestens einer Stimulation und/oder Messung in einem menschlichen oder tierischen Körper, beispielsweise für einen Herzschrittmacher, mit mindestens einem Bündel (3, 23, 43, 63) von Drähten, dadurch gekennzeichnet, dass die Drähte durch Fasern (5, 25, 45, 65) mit einer weniger als 20 Mikrometer betragenden Dicke gebildet sind.

2. Kabel nach Anspruch 1, dadurch gekennzeichnet, dass die Dicke der im Querschnitt rundlichen, vorzugsweise kreisförmigen Fasern (5, 25, 45, 65) höchstens 15 Mikrometer beträgt.

3. Kabel nach Anspruch 1 oder 2, dadurch gekennzeichnet, dass das bzw. jedes Bündel mindestens 100, zweckmässigerweise mindestens 200, vorzugsweise mindestens 500 und beispielsweise mindestens 1000 Fasern (5, 25, 45, 65) aufweist.

4. Kabel nach einem der Ansprüche 1 bis 3, dadurch gekennzeichnet, dass die Fasern (5, 25, 45, 65) mindestens im allgemeinen derart schief zur Längsrichtung des Kabels verlaufen, dass jede Faser (5, 25, 45, 65) höchstens 50% und zum Beispiel höchstens oder ungefähr 30% länger ist als das Kabel.

5. Kabel nach einem der Ansprüche 1 bis 4, dadurch gekennzeichnet, dass die Fasern (5, 25, 45, 65) um die Längsachse des von ihnen gebildeten Bündels (3, 23, 43, 63) herum gedreht sind und/oder dass das bzw. jedes Bündel (3, 23) mindestens stellenweise mit der Längsrichtung des Kabels einen Winkel bildet und zum Beispiel zickzack- und/oder wellen-und/oder wendelförmig ist, wobei zum Beispiel mehrere Bündel (3, 23) durch Flechten, Wirken, Weben oder Zusammendrehen miteinander verbunden sind.

6. Kabel nach einem der Ansprüche 1 bis 5, dadurch gekennzeichnet, dass mindestens die zum gleichen Bündel (3, 23, 43, 63) gehörenden und einander benachbarten Fasern (5, 25, 45, 65) einander zumindest stellenweise berühren, wobei das bzw. jedes Bündel (3, 23, 43, 63) vorzugsweise zumindest auf der Aussenseite des Kabels durch eine gummielastische Isolation (7, 27, 47, 67) elastisch isoliert und zum Beispiel in den Mantel einer schlauchförmigen Isolation (7, 27, 47) eingebettet ist, die eine zentrale Längsöffnung (9, 29, 49) begrenzt.

7. Kabel nach einem der Ansprüche 1 bis 6, gekennzeichnet durch mindestens zwei Faser-Bündel (43) und/oder Gruppen von Faser-Bündeln (23), die elektrisch gegeneinander isoliert sind.

8. Kabel nach einem der Ansprüche 1 bis 7, dadurch gekennzeichnet, dass das metallische Material der Fasern (5, 25, 45, 65) zum Teil aus Titan, vorzugsweise einer Legierung des alpha-beta-Typs oder des beta-Typs, besteht, wobei das Titan vorzugsweise den gewichtsmässig grössten, zum Beispiel mindestens 50 Gew.-% betragenden Anteil an der Legierung hat und wobei die Fasern beispielsweise mit einer Schicht Metalloxyd überzogen sind.

9. Kabel nach Anspruch 8, dadurch gekennzeichnet, dass die Fasern (5, 25, 45, 65) zusätzlich zu Titan noch mindestens eines der Metalle Niob, Tantal, Zirkon, Chrom, Molybdän, Eisen und Aluminium enthalten.

10. Verfahren zur Herstellung eines Kabels nach einem der Ansprüche 1 bis 9, dadurch gekennzeichnet, dass mehrere aus dem Material der zu bildenden Fasern (5, 25, 45, 65) bestehende, dickere Drähte in eine aus einem andern metallischen Material bestehende Matrix eingebettet und zusammen mit dieser durch Verformen länger sowie dünner gemacht werden und dass danach die Matrix mit einer Säure herausgelöst wird, so dass ein Bündel Fasern (5, 25, 45, 65) entsteht.

11. Verfahren nach Anspruch 10, dadurch gekennzeichnet, dass zumindest der Schlussteil der zur Bildung der Fasern (5, 25, 45, 65) durch Verlängern und Querschnittsflächenverminderung vorgenommenen Verformung eine Kaltverformung ist und die Fasern (5, 25, 45, 65) nach dieser Kaltverformung einer Wärmebehandlung, wie Glühen, zum Beispiel Weichglühen oder Lösungsglühen, und/oder Auslagern, unterzogen werden.

12. Verfahren nach Anspruch 10 oder 11, dadurch gekennzeichnet, dass die Fasern anodisch oxydiert werden.

Fig. 1

Fig. 2

Fig. 3

# Fig. 4

# Fig. 5